# EUROPEAN PATENT APPLICATION

(11) **EP 4 670 777 A1**
(43) Date of publication of application: **31.12.2025**
(21) Application number: 24848222.6
(22) Date of filing: 29.07.2024
(51) Int. Cl.: A61M 60/178, A61M 60/126, A61M 60/122, A61M 60/237, A61M 60/802

(54) **CATHETER PUMP**

(30) Priority: 31.07.2023 CN 202322037299 U
(71) Applicant: Magassist Co., Ltd., Suzhou, Jiangsu 215163 (CN)
(72) Inventor: TAN, Renmu, Suzhou, Jiangsu 215163 (CN)
(74) Representative: Grünecker Patent- und Rechtsanwälte PartG mbB
(86) International application number: PCT/CN2024/108126
(87) International publication number: WO 2025/026267

(57) **Abstract**

A catheter pump, comprising: a catheter and a pump head located at the distal end of the catheter and used for pumping blood. The pump head comprises a pump housing provided with a blood inlet and a blood outlet; the pump housing comprises a stent and a graft that are connected to the distal end of the catheter; the stent is operably switched between a radial folded state and a radial unfolded state; in the radial unfolded state, the stent comprises a body portion covered with the graft, an inlet portion located at the axial distal end of the body portion and an outlet portion located at the axial proximal end of the body portion; the inlet portion and the outlet portion are connected to and support the body portion; a distal end connecting portion is connected to the distal end of the inlet portion; and the graft comprises a first connecting portion and a second connecting portion located at the distal end thereof, the second connecting portion is connected to the distal end of the first connecting portion, at least part of the first connecting portion is located on the outer surface of the inlet portion, the second connecting portion is located on the outer surface of the distal end connecting portion and is fixedly connected to the distal end connecting portion, and the circumferential width of the second connecting portion is greater than that of the first connecting portion.

## Description

The present application claims the priority to Chinese Patent Application No. 202322037299.5, titled "CATHETERPUMP", filed on July 31, 2023 with the China National Intellectual Property Administration, which is incorporated herein by reference in its entirety.

### FIELD

The present application relates to the technical field of medical devices, and in particular to a catheter pump.

### BACKGROUND

Catheter pumps are categorized into non-collapsible and collapsible types. The collapsible catheter pump creates a smaller incision wound during interventional procedures, offering the advantage of more convenient and rapid use.

The collapsible catheter pump features a collapsible pump head, which includes: a pump housing defined by a film and a stent; and an impeller located inside the stent. The stent generally includes a centrally located, generally cylindrical pump portion, and an inlet portion and an outlet portion that are generally conical in shape and located at axial ends of the pump portion respectively. The inlet portion at a distal end of the stent is positioned outside a distal end of a film body of the film in an axial direction, with the stent supporting and maintaining a shape of the distal end of the film to expand the film. The film may be provided on the stent and fixedly connected to the stent by adhesive bonding for example. By applying constraints to the distal end of the film via the stent, it expands or collapses along with the stent, preventing separation between the two.

However, the collapse of the distal end of the stent may easily lead to the detachment of the adhered film. Increasing a coverage area of the film over the distal end of the stent may enhance the connection stability between the film and the stent, making the film less prone to detachment. Nevertheless, an excessively large coverage area may reduce the size of the blood inlet, thereby affecting the blood-pumping function of the catheter pump.

### SUMMARY

In view of the deficiencies in the conventional technology, an object of the present application is to provide a catheter pump that ensures the film is less likely to detach without affecting the blood inlet.

A catheter pump includes a catheter and a pump head located at a distal end of the catheter for pumping blood. The pump head includes a pump housing with at least one blood inlet and at least one blood outlet. The pump housing includes a stent and a film, and the stent is connected to the distal end of the catheter and is operatively switchable between a radially collapsed state and a radially expanded state. In the radially expanded state, the stent includes a main body portion covered by the film, an inlet portion located at a distal end of the main body portion in an axial direction, and an outlet portion located at a proximal end of the main body portion in the axial direction. The inlet portion and the outlet portion are connected to and support the main body portion. A distal connecting portion is connected to a distal end of the inlet portion. The film includes a first connecting portion and a second connecting portion that are located at a distal end of the film. The second connecting portion is connected to a distal end of the first connecting portion. At least part of the first connecting portion is positioned on an outer surface of the inlet portion, and the second connecting portion is located on an outer surface of the distal connecting portion and fixedly connected to the distal connecting portion. A width of the second connecting portion in a circumferential direction is greater than a width of the first connecting portion in the circumferential direction.

In an example, the inlet portion includes multiple connecting struts connected between the main body portion and the distal connecting portion, and the width of the first connecting portion in the circumferential direction is less than or equal to a maximum width of the connecting struts in the circumferential direction.

In an example, the second connecting portion is connected to the distal connecting portion in such a manner that the second connecting portion does not overlap itself.

In an example, the number of the first connecting portion is equal to that of the second connecting portion, and there are multiple first connecting portions.

In an example, the film includes a cylindrical section, with a distal end of the cylindrical section being connected to the first connecting portion, and the cylindrical section covers an outer surface of the main body portion. The multiple first connecting portions are uniformly distributed in a circumferential direction of the cylindrical section.

In an example, multiple second connecting portions have a same length in the axial direction and/or a same width in the circumferential direction.

In an example, the multiple first connecting portions have a same length in the axial direction and are all located on the outer surface of the inlet portion. The multiple second connecting portions are arranged in sequence in the circumferential direction and fixedly connected to the outer surface of the distal connecting portion.

In an example, a sum of the widths of the multiple second connecting portions in the circumferential direction is approximately equal to a width of the distal connecting portion in the circumferential direction.

In an example, a length of each second connecting portion in the axial direction is equal to a length of the distal connecting portion in the axial direction.

In an example, lengths of the multiple first connecting portions in the axial direction are not equal, with part of the multiple first connecting portions being located on the outer surface of the distal connecting portion. The multiple second connecting portions are arranged in sequence in the axial direction and fixedly connected to the outer surface of the distal connecting portion.

In an example, a sum of the lengths of the multiple second connecting portions in the axial direction is approximately equal to a length of the distal connecting portion in the axial direction.

In an example, the width of each second connecting portion in the circumferential direction is greater than half of a width of the distal connecting portion in the circumferential direction and less than the width of the distal connecting portion in the circumferential direction.

In an example, the distal connecting portion includes at least one connecting leg, and is connected to a distal bearing housing, with an outer wall surface of the distal bearing housing having at least one groove for accommodating the connecting leg in one-to-one correspondence. The second connecting portion is fixed on the outer wall surface of the distal bearing housing.

In an example, a distal collar is provided on the second connecting portion and is configured to radially fix the connecting leg and fix the second connecting portion to the outer surface of the distal connecting portion.

In an example, a total width of the first connecting portions in the circumferential direction is 1/5 to 1/20 of a maximum circumference of the inlet portion.

In an example, the total width of the first connecting portions in the circumferential direction is 1/5 to 1/10 of the maximum circumference of the inlet portion.

In the catheter pump provided in this embodiment, the first connecting portion and the second connecting portion connected to each other are provided at the distal end of the film. At least part of the first connecting portion is located on the outer surface of the inlet portion of the stent, and the second connecting portions is located on the outer surface of the distal connecting portion of the stent and fixedly connected to the distal connecting portion. The second connecting portion enhances the connection strength between the film and the stent, effectively preventing the film from detaching when the distal end of the stent is collapsed. Meanwhile, the first connecting portion minimizes the coverage area of the film over the inlet portion, thus not affecting the blood inlet.

### BRIEF DESCRIPTION OF THE DRAWINGS

FIG. 1 is a schematic structural view of a film provided according to an embodiment of the present application before mounting;
FIG. 2 is a schematic structural view of the film in FIG. 1 after mounting;
FIG. 3 is a schematic structural view of a film provided according to another embodiment of the present application before mounting;
FIG. 4 is a schematic structural view of the film in FIG. 3 after mounting;
FIG. 5 is a schematic structural view of a film provided according to an embodiment of the present application mounted on a stent;
FIG. 6 is a schematic structural view of FIG. 5 from another perspective;
FIG. 7 is a schematic structural view of a stent provided according to an embodiment of the present application in a radially expanded state;
FIG. 8 is a schematic structural view of a catheter pump provided according to another embodiment of the present application;
FIG. 9 is a partial cross-sectional view of FIG. 8.

Reference numerals in the drawings are as follows:

| | | | |
|---|---|---|---|
| 1000. | catheter pump; | 100. | power assembly; |
| 101. | shell; | 200. | working assembly; |
| 201. | catheter; | 202. | driving shaft; |
| 2021. | flexible shaft; | 2022. | rigid shaft; |
| 204. | driving catheter handle; | 205. | pump head; |
| 2051. | pump housing; | 2051a. | blood inlet; |
| 2051b. | blood outlet; | 20511. | stent; |
| 20512. | film; | 2052. | impeller; |
| 20521. | hub; | 206. | proximal bearing housing; |
| 207. | distal bearing housing; | 208. | proximal bearing; |
| 209. | distal bearing; | 210. | atraumatic support member; |
| 73. | distal collar; | 11. | main body portion; |
| 12. | inlet portion; | 121. | connecting strut; |
| 13. | outlet portion; | 14. | proximal connecting portion; |
| 15. | distal connecting portion; | 16. | first connecting portion; |
| 17. | second connecting portion; | 18. | cylindrical section; |
| X. | axial direction. | | |

### DETAILED DESCRIPTION OF THE EMBODIMENTS

The terms such as "proximal", "rear", "distal", and "front" as used in the present application are defined relative to the physician operating the catheter pump. The terms "proximal" and "rear" refer to the portion that is relatively closer to the physician, while the terms "distal" and "front" refer to the portion that is relatively farther away from the physician. For example, the portion outside the body is located at the proximal or rear end, and the portion inserted into the body is located at the distal or front end. For the catheter pump in the present application, "axial" or "axial extension direction" is defined based on an extension direction of the driving shaft. The terms "inner" and "outer" are defined relative to the central axis of the axial extension: specifically, the direction relatively closer to the central axis is referred to as "inner", and the direction relatively farther away from the central axis is referred to as "outer".

It should be understood that the orientation terms such as "proximal", "distal", "rear", "front", "inner", and "outer" are defined for the convenience of description. However, the catheter pump may be used in many directions and positions, so these terms expressing relative positional relationships are not restrictive or absolute. For instance, the aforementioned definitions of orientations are merely for the convenience of explaining the technical solutions of the present application and do not limit the orientation of the catheter pump of the present application in other scenarios where inversion or positional changes may occur, including but not limited to product testing, transportation, and manufacturing. In the present application, if there are explicit definitions and limitations for the aforementioned terms, they should comply with those explicit definitions and limitations above.

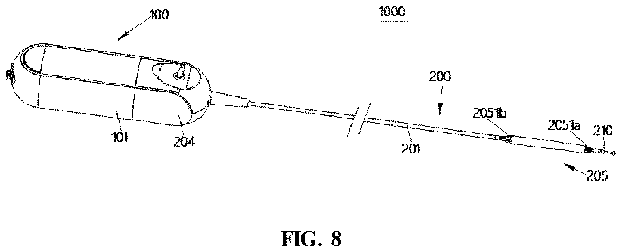

Referring to FIGS. 8 to 9, a catheter pump 1000 according to an embodiment of the present application may be at least partially inserted into a subject to assist the pumping function of the heart and reduce cardiac workload. In scenarios suitable for left ventricular assistance, the catheter pump 1000 pumps blood from the left ventricle into the aorta, providing support for blood circulation, reducing the workload on the heart of the subject, or offering additional continuous pumping power support when the pumping capacity of the heart is insufficient. Certainly, the catheter pump 1000 may also be inserted into other desired target locations of the subject, such as the right ventricle, blood vessels, or other organs, through interventional procedures.

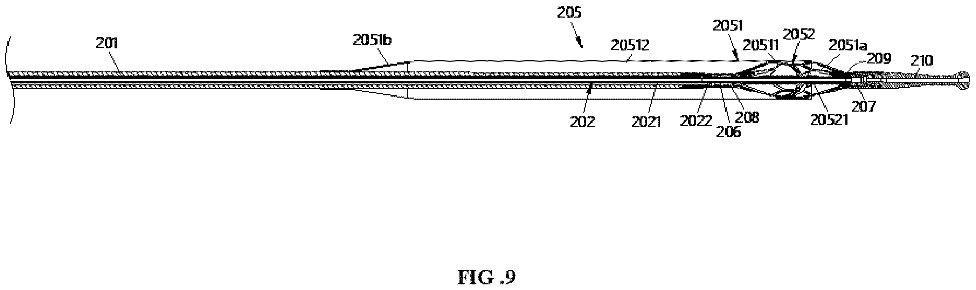

As shown in FIG. 8, the catheter pump 1000 includes a power assembly 100 and a working assembly 200. The power assembly 100 includes a shell 101, a motor (not shown) housed within the shell 101, and a driving member (not shown) driven by the motor. As illustrated in FIG. 9, the working assembly 200 includes: a catheter 201, a driving shaft 202 extending through the catheter 201, a driven member connected to a proximal end of the driving shaft 202, as well as a driving catheter handle 204 connected to a proximal end of the catheter 201 and a pump head 205 connected to a distal end of the catheter 201. The pump head 205 may be delivered to a desired location in the heart, such as the left ventricle, via the catheter 201 to pump blood. The pump head 205 includes a pump housing 2051 with at least one blood inlet 2051a and at least one blood outlet 2051b, and an impeller 2052 housed within the pump housing 2051. The blood inlet 2051a is located at a distal end of the pump housing 2051, and the blood outlet 2051b is located at a proximal end of the pump housing 2051. The motor is positioned at the proximal end of the catheter 201 and drives the impeller 2052 to rotate for pumping blood via the driving shaft 202. The impeller 2052 is connected to a distal end of the driving shaft 202. When the impeller 2052 rotates, it draws blood into the pump housing 2051 through the blood inlet 2051a and then pumps blood out of the pump housing 2051 through the blood outlet 2051b.

The pump housing 2051 is connected to the distal end of the catheter 201, and the impeller 2052 is connected to the distal end of the driving shaft 202. The pump housing 2051 includes a film 20512 that defines a blood flow channel, and a collapsible stent 20511 that supports and expands the film 20512. A proximal end of the stent 20511 is connected to the distal end of the catheter 201. In other embodiments, the stent may alternatively be in other forms of expansion mechanisms that are configured to expand and maintain a fixed shape and cooperate with the film to form the corresponding blood inlet.

The film 20512 is elastic and covers a portion of the stent 205 11, leaving another portion uncovered to serve as the blood inlet or the blood outlet. The impeller 2052 is housed within the stent 20511 and positioned inside the film 20512.

A distal bearing housing 207 is provided at a distal end of the stent 20511, and a distal bearing 209 is disposed in the distal bearing housing 207 to rotationally support the distal end of the driving shaft 202. The driving shaft 202 includes a bendable flexible shaft 2021 extending through the catheter 201, and a rigid shaft 2022 connected to a distal end of the flexible shaft 2021 and extending through a hollow channel in a hub 20521 of the impeller 2052. The hub 20521 of the impeller 2052 is arranged on the rigid shaft 2022, with proximal and distal ends of the rigid shaft 2022 passing through a proximal bearing 208 and the distal bearing 209, respectively. In this way, the rigid shaft 2022 is supported at both ends thereof by the two bearings, and combined with its high rigidity, it provides rigid support for the impeller 2052 inside the pump housing 2051, ensuring the impeller 2052 is optimally positioned inside the pump housing 2051 and maintains a stable position therein.

At a distal end of the distal bearing housing 207, there is an atraumatic support member 210 made of a flexible material. The atraumatic support member 210 is supported against an inner wall of the ventricle in an atraumatic or non-invasive manner, separating the blood inlet 2051a of the pump head 205 from the inner wall of the ventricle, which prevents the blood inlet 2051a of the pump head 205 from adhering to the inner wall of the ventricle due to a reactive force of the blood during the operation of the pump head 205, thus ensuring an effective pumping area.

The driving catheter handle 204 is detachably connected to the power assembly 100, and the connection between them may be achieved by a locking nut, or a snap-fit connection as described in US9421311B2. The driven member is coupled to the driving member in a non-contact manner to transmit the rotational power of the motor to the driving shaft 202, thereby driving the impeller 2052 to rotate and pump blood. As mentioned above, the coupling between the driven member and the driving member may adopt a magnetic coupling as described in CN103120810B or CN101820933B, or eddy current coupling as described in CN216061675U or CN114452527A, which will not be limited in this embodiment.

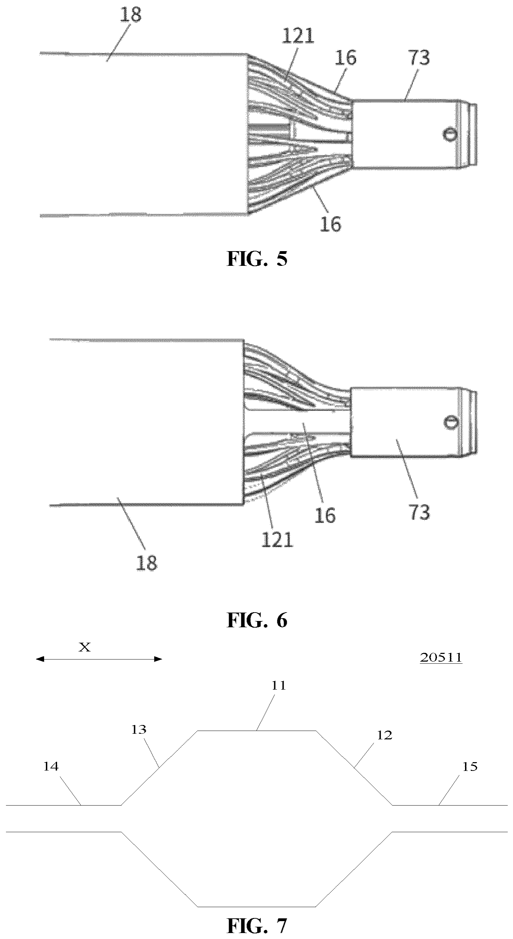

Referring to FIG. 7, the stent 20511 in the embodiment of the present application may be operatively switched between a radially collapsed state and a radially expanded state. In the radially expanded state, the stent 20511 includes a generally cylindrical main body portion 11 covered by the film 20512, and a generally conical inlet portion 12 and outlet portion 13 located at distal and proximal ends of the main body portion 11 in an axial direction X, respectively. The inlet portion 12 and the outlet portion 13 are connected to and support the main body portion 11. The blood inlet 2051a is formed at the location of the inlet portion 12. A proximal connecting portion 14 is connected to a proximal end of the outlet portion 13, and a distal connecting portion 15 is connected to a distal end of the inlet portion 12.

The proximal connecting portion 14 and the distal connecting portion 15 are configured to connect the stent 20511 to other components at the proximal and distal ends, respectively. For example, the proximal connecting portion 14 may connect the stent 20511 to a proximal bearing housing 206 or the catheter 201, and the distal connecting portion 15 may connect the stent 20511 to the distal bearing housing 207 or the atraumatic support member 210.

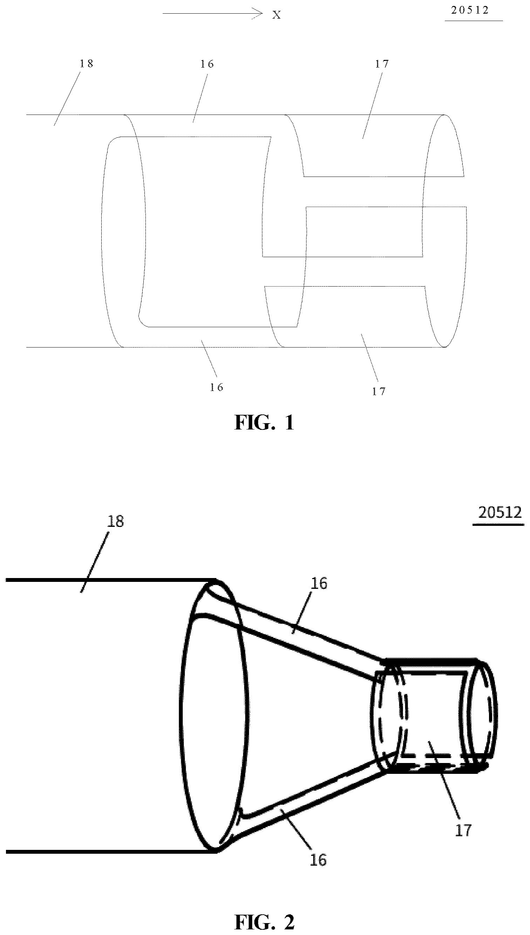

Referring to FIGS. 1 to 4, in this embodiment, the film 20512 includes a first connecting portion 16 and a second connecting portion 17 that are located at a distal end of the film. The second connecting portion 17 is connected to a distal end of the first connecting portion 16. At least part of the first connecting portion 16 is located on an outer surface of the inlet portion 12, and the second connecting portion 17 is located on an outer surface of the distal connecting portion 15 and is fixedly connected to the distal connecting portion 15. Thus, the second connecting portion 17 may increase the connection strength between the film 20512 and the stent 20511, effectively preventing the film 20512 from detaching when the distal end of the stent 20511 is collapsed. Meanwhile, the first connecting portion 16 minimizes the coverage area of the film 20512 over the inlet portion 12, thereby not affecting the blood inlet 2051a. Specifically, the second connecting portion has an opening. During mounting, the first connecting portion 16 is first attached to the stent, and then the second connecting portion 17 at the distal end is wrapped around the outer surface of the distal connecting portion 15 and is fixedly connected to the distal connecting portion 15, achieving a fixed connection conveniently.

Specifically, the film 20512 further includes a cylindrical section 18, with a distal end of the cylindrical section 18 being connected to the first connecting portion 16. The cylindrical section 18 covers an outer surface of the main body portion 11, and the first connecting portion 16 is connected to the distal end of the cylindrical section 18. When the stent 20511 is in the radially expanded state, the first connecting portion 16 located on the outer surface of the inlet portion 12 is generally conical in shape. A proximal end of the cylindrical section 18 is positioned outside the catheter 201 and fixed to an outer wall of the catheter 201.

In this embodiment, the stent 20511 is supported at the distal end of the film 20512, with a part of the stent 20511 being located outside the distal end of the film 20512 and another part of the stent 20511 being located inside the film 20512. The film 20512 covers a middle portion and a rear portion of the stent 205 11, and the mesh of the part at a front portion of the stent 20511 that is uncovered by the film 20512 forms the blood inlet 2051a. A rear end of the film 20512 is covered over an outer surface of the distal end of the catheter 201, and the blood outlet 2051b is the opening formed at the rear end of the film 20512.

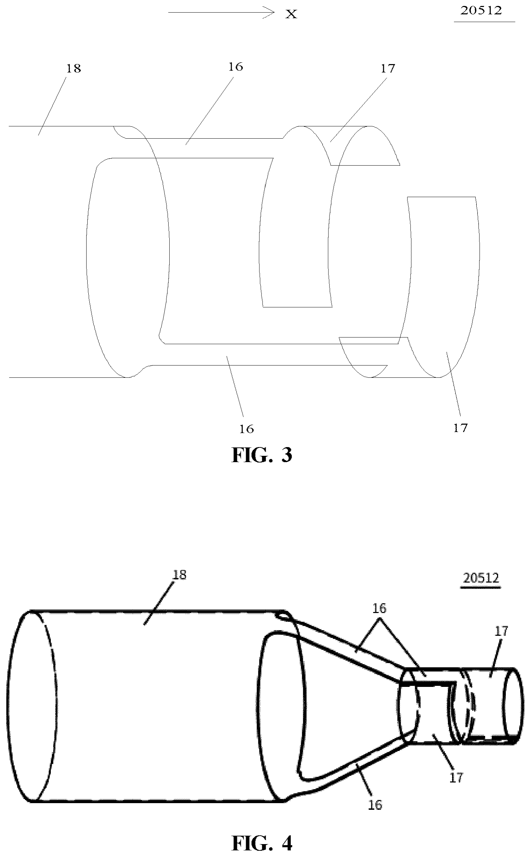

As shown in FIGS. 1 and 3, a width of the second connecting portion 17 in a circumferential direction is greater than a width of the first connecting portion 16 in the circumferential direction. Since at least part of the first connecting portion 16 is located on the outer surface of the inlet portion 12, reducing the width of the first connecting portion 16 in the circumferential direction may decrease the coverage area of the film 20512 over the blood inlet 2051a, while increasing the width of the second connecting portion 17 in the circumferential direction may further enhance the connection strength between the film 20512 and the stent 20511. Specifically, as shown in FIGS. 5 and 6, the inlet portion 12 includes multiple connecting struts 121 that are connected between the main body portion 11 and the distal connecting portion 15. The width of the first connecting portion 16 in the circumferential direction is less than or equal to a maximum width of the connecting struts 121 in the circumferential direction, minimizing the coverage of the first connecting portion 16 over the blood inlet 2051a.

In this embodiment, the second connecting portion 17 is connected to the distal connecting portion 15 in such a manner that the second connecting portion 17 does not overlap itself, meaning that at most one layer of the second connecting portion 17 is located on the outer surface of the distal connecting portion 15. This prevents excessive thickness of the second connecting portion 17, thereby facilitating a reduction in the size of the catheter pump 1000 and enabling its miniaturization. In other embodiments, the second connecting portion may be wrapped on the distal connecting portion 15 in an integer number of turns when it connected to the distal connecting portion 15, minimizing unnecessary overlapping of the second connecting portion as much as possible.

In other embodiments, exactly two layers of the second connecting portions 17 are fixedly provided on the outer surface of the distal connecting portion 15, without adding more than two layers of thickness, while still increasing the connection strength between the film 20512 and the stent 20511.

In this embodiment, the number of the first connecting portion 16 is equal to that of the second connecting portion 17, with a distal end of each first connecting portion 16 being connected to one corresponding second connecting portion 17. To ensure uniform force distribution across the film 20512, multiple first connecting portions 16 are provided, for example, two, three, four or more first connecting portions 16 are provided. In an embodiment, the multiple first connecting portions 16 are uniformly distributed in a circumferential direction of the cylindrical section 18, which ensures that the multiple second connecting portions 17 are uniformly connected to the cylindrical section 18, thereby uniformly increasing the connection strength between the film 20512 and the stent 20511 at various locations.

In an embodiment, the multiple second connecting portions 17 have a same length in the axial direction X and/or a same width in the circumferential direction. When the multiple second connecting portions 17 have the same length in the axial direction X and the same width in the circumferential direction, the increase in connection strength between the film 20512 and the stent 20511 contributed by each of the second connecting portions 17 is equal

Specifically, in this embodiment, the first connecting portion 16 features a strip-shaped structure with a constant width, enabling it to maintain stable connection strength. A total width of the first connecting portions 16 in the circumferential direction is 1/10 of a maximum circumference of the inlet portion 12. In other embodiments, the total width of the first connecting portions 16 in the circumferential direction is 1/5 to 1/20 of the maximum circumference of the inlet portion 12.

In other embodiments, the width of the first connecting portion 16 may vary in accordance with the dimensional variation of the inlet portion 12 from its proximal end to the distal end, i.e., gradually tapering from the proximal end to the distal end thereof. However, the proportion of the total width of the first connecting portion 16 in the circumferential direction to the circumference of the inlet portion 12 at the corresponding position remains constant, meaning the opening degree of the inlet portion remains unchanged.

In an embodiment, as shown in FIGS. 1 and 2, the multiple first connecting portions 16 have a same length in the axial direction X and are all located on the outer surface of the inlet portion 12. The multiple second connecting portions 17 are arranged in sequence in the circumferential direction and fixedly connected to the outer surface of the distal connecting portion 15. This structure is simple, as it is unnecessary to extend the first connecting portions 16 onto the outer surface of the distal connecting portion 15, simplifying the structure of the film 20512 while ensuring that the film 20512 is not prone to detachment and does not interfere with the blood inlet 2051a.

In one embodiment, a sum of the widths of the multiple second connecting portions 17 in the circumferential direction is approximately equal to a width of the distal connecting portion 15 in the circumferential direction. Here, the term "approximately equal" encompasses two situations. In one situation, the sum of the widths of the multiple second connecting portions 17 in the circumferential direction may be equal to the width of the distal connecting portion 15 in the circumferential direction, so that the multiple second connecting portions 17 exactly encircle the distal connecting portion 15 for one turn, utilizing the total width of the distal connecting portion 15 in the circumferential direction to increase the connection strength between the film 20512 and the stent 20511. In the other situation, the sum of the widths of the multiple second connecting portions 17 in the circumferential direction may be slightly less than the width of the distal connecting portion 15 in the circumferential direction, and a gap may be maintained between every two adjacent of the second connecting portions 17 in the circumferential direction. This arrangement ensures that the second connecting portions 17 do not overlap with each other in the circumferential direction, avoiding an increase in the size of the catheter pump 1000 due to excessive thickness of the second connecting portions 17.

In a further embodiment, the length of the second connecting portion 17 in the axial direction X is equal to the length of the distal connecting portion 15 in the axial direction X, so that the second connecting portion 17 may fully cover the distal connecting portion 15 in the axial direction X, utilizing the total length of the distal connecting portion 15 in the circumferential direction to increase the connection strength between the film 20512 and the stent 20511.

In another embodiment, as shown in FIGS. 3 and 4, lengths of the multiple first connecting portions 16 in the axial direction X are not equal, with part of the first connecting portions 16 extending onto the outer surface of the distal connecting portion 15. The multiple second connecting portions 17 are arranged in sequence in the axial direction X and fixedly connected to the outer surface of the distal connecting portion 15. Thus, each of the second connecting portions 17, together with the first connecting portions 16 located on the outer surface of the distal connecting portion 15, forms a nested structure, which may further increase the connection strength between the film 20512 and the stent 20511.

In an embodiment, a sum of the lengths of the multiple second connecting portions 17 in the axial direction X is approximately equal to the length of the distal connecting portion 15 in the axial direction X. Here, the term "approximately equal" encompasses two situations. In one situation, the sum of the lengths of the multiple second connecting portions 17 in the axial direction X may be equal to the length of the distal connecting portion 15 in the axial direction X, so that the multiple second connecting portions 17 precisely and fully cover the distal connecting portion 15 in the axial direction X, utilizing the total length of the distal connecting portion 15 in the axial direction X to enhance the connection strength between the film 20512 and the stent 20511. In the other situation, the sum of the lengths of the multiple second connecting portions 17 in the axial direction X may be slightly less than the length of the distal connecting portion 15 in the axial direction X, and a gap may be maintained between every two adjacent of the second connecting portions 17 in the axial direction X. This arrangement ensures that the second connecting portions 17 do not overlap with each other in the axial direction X, preventing an increase in the size of the catheter pump 1000 due to excessive thickness of the second connecting portions 17.

In an embodiment, the width of each second connecting portion 17 in the circumferential direction is greater than half of a width of the distal connecting portion 15 in the circumferential direction and less than the width of the distal connecting portion 15 in the circumferential direction. When there are two first connecting portions 16, the sum of the widths of one first connecting portion 16 and one second connecting portion 17 in the circumferential direction is approximately equal to the width of the distal connecting portion 15 in the circumferential direction, thereby fully utilizing the entire width of the distal connecting portion 15 in the circumferential direction to enhance the connection strength between the film 20512 and the stent 20511.

In this embodiment, the distal connecting portion 15 may include at least one connecting leg (not shown in the figure). The distal connecting portion 15 is connected to a distal bearing housing 207, and an outer wall surface of the distal bearing housing 207 is provided with at least one groove (not shown in the figure) for accommodating the connecting leg in one-to-one correspondence, ensuring a reliable and stable connection between the stent 20511 and the distal bearing housing 207. The second connecting portion 17 is fixed on the outer wall surface of the distal bearing housing 207, and is fixedly connected to the distal bearing housing 207 together with the distal connecting portion 15.

Specifically, a distal collar 73 is provided on the second connecting portion 17 and is configured to radially fix and connect the connecting leg, keeping the connecting leg consistently positioned in the groove. The distal collar 73 is configured to fix the second connecting portion 17 to the outer surface of the distal connecting portion 15, achieving a fixed connection between the second connecting portion 17 and the distal connecting portion 15, and ensuring that the second connecting portion 17 enhances the connection strength between the film 20512 and the stent 20511.

The foregoing embodiments only describe some implementations of the present application, which are specifically described in detail and are not construed as limitation to the scope of the present application. It should be noted that for those skilled in the art, variations and improvements can be made without departing from the concept of the present application, which fall within the protection scope of the present application. Hence, the protection scope of the present application is in accordance with the appended claims.

## Claims

1. A catheter pump, comprising a catheter and a pump head located at a distal end of the catheter for pumping blood; wherein
the pump head comprises a pump housing with at least one blood inlet and at least one blood outlet;
the pump housing comprises a stent and a film, the stent is connected to the distal end of the catheter and is operably switchable between a radially collapsed state and a radially expanded state;
in the radially expanded state, the stent comprises a main body portion covered by the film, an inlet portion located at an a distal end of the main body portion in an axial direction, and an outlet portion located at a proximal end of the main body portion in the axial direction, wherein the inlet portion and the outlet portion are connected to and support the main body portion, and a distal connecting portion is connected to a distal end of the inlet portion; and
the film comprises a first connecting portion and a second connecting portion which are located at a distal end of the film, the second connecting portion is connected to a distal end of the first connecting portion, at least part of the first connecting portion is located on an outer surface of the inlet portion, the second connecting portion is located on an outer surface of the distal connecting portion and is fixedly connected to the distal connecting portion, and a width of the second connecting portion in a circumferential direction is greater than a width of the first connecting portion in the circumferential direction.

2. The catheter pump according to claim 1, wherein the inlet portion comprises a plurality of connecting struts connected between the main body portion and the distal connecting portion, and the width of the first connecting portion in the circumferential direction is less than or equal to a maximum width of the connecting struts in the circumferential direction.

3. The catheter pump according to claim 1, wherein the second connecting portion is connected to the distal connecting portion in such a manner that the second connecting portion does not overlap itself.

4. The catheter pump according to claim 1, wherein the number of the first connecting portion is equal to that of the second connecting portion, and there are a plurality of first connecting portions.

5. The catheter pump according to claim 4, wherein the film comprises a cylindrical section, with a distal end of the cylindrical section being connected to the first connecting portion, and the cylindrical section covers an outer surface of the main body portion;
the plurality of first connecting portions are uniformly distributed in a circumferential direction of the cylindrical section; and
preferably, a plurality of second connecting portions have a same length in the axial direction and/or a same width in the circumferential direction.

6. The catheter pump according to claim 4, wherein the plurality of first connecting portions have a same length in the axial direction and are all located on the outer surface of the inlet portion, and the plurality of second connecting portions are arranged in sequence in the circumferential direction and fixedly connected to the outer surface of the distal connecting portion.

7. The catheter pump according to claim 6, wherein a sum of the widths of the plurality of second connecting portions in the circumferential direction is approximately equal to a width of the distal connecting portion in the circumferential direction, and
preferably, a length of each second connecting portion in the axial direction is equal to a length of the distal connecting portion in the axial direction.

8. The catheter pump according to claim 4, wherein lengths of the plurality of first connecting portions in the axial direction are not equal, part of the plurality of first connecting portions are located on the outer surface of the distal connecting portion, and the plurality of second connecting portions are arranged in sequence in the axial direction and fixedly connected to the outer surface of the distal connecting portion.

9. The catheter pump according to claim 8, wherein a sum of lengths of the plurality of second connecting portions in the axial direction is approximately equal to a length of the distal connecting portion in the axial direction, and
preferably, the width of each second connecting portion in the circumferential direction is greater than half of a width of the distal connecting portion in the circumferential direction and less than or equal to the width of the distal connecting portion in the circumferential direction.

10. The catheter pump according to claim 1, wherein the distal connecting portion comprises at least one connecting leg, the distal connecting portion is connected to a distal bearing housing, and an outer wall surface of the distal bearing housing is provided with at least one groove for accommodating the connecting leg in one-to-one correspondence;
the second connecting portion is fixed on the outer wall surface of the distal bearing housing; and
a distal collar is provided on the second connecting portion and is configured to radially fix the connecting leg and fix the second connecting portion to the outer surface of the distal connecting portion.

11. The catheter pump according to claim 1, wherein a total width of the first connecting portion in the circumferential direction is 1/5 to 1/20 of a maximum circumference of the inlet portion; and
preferably, the total width of the first connecting portion in the circumferential direction is 1/5 to 1/10 of the maximum circumference of the inlet portion.
